Europäisches Patentamt

(19) European Patent Office

Office européen des brevets

(11) EP 0 499 942 B1

(12) **EUROPEAN PATENT SPECIFICATION**

(45) Date of publication and mention
of the grant of the patent:
**27.08.1997 Bulletin 1997/35**

(51) Int. Cl.⁶: **B31F 1/07**

(21) Application number: **92102221.6**

(22) Date of filing: **10.02.1992**

(54) **Embossed web, method and apparatus for making same**

Geprägtes Band, Verfahren und Vorrichtung zur dessen Herstellung

Bande gaufrée, procédé et dispositif pour sa fabrication

(84) Designated Contracting States:
**BE DE ES FR GB IT NL SE**

(30) Priority: **22.02.1991 US 660317**

(43) Date of publication of application:
**26.08.1992 Bulletin 1992/35**

(73) Proprietor: **KIMBERLY-CLARK CORPORATION**
**Neenah, Wisconsin 54956-0349 (US)**

(72) Inventors:
• **Veith, Jerome Steven**
  **Menasha, Wisconsin 54952 (US)**
• **Grupe, Edward Howard**
  **Appleton, Wisconsin 54914 (US)**

(74) Representative: **Diehl, Hermann O. Th., Dr. et al**
**Diehl, Glaeser, Hiltl & Partner**
**Patentanwälte**
**Postfach 19 03 65**
**80603 München (DE)**

(56) References cited:
EP-A- 0 136 368          EP-A- 0 408 248
GB-A- 1 549 218          US-A- 2 834 809
US-A- 3 240 657          US-A- 3 708 366

• PATENT ABSTRACTS OF JAPAN vol. 8, no. 201
(M-325)(1638) 14 September 1984

## Description

The invention refers to an embossed web, method and apparataus for making same.

In the manufacture of tissue products such as facial tissue, bath tissue and paper towels, it is known that embossing increases the bulk of the product as well as improves aesthetic appeal. A trade-off, however, is the fact that embossing also reduces the strength of the embossed sheet and, in the case of roll products, an increase in bulk is accompanied by a decrease in roll firmness.

US-A-2,834,809 describes a method of embossing paper using a patterned roller, in which the clearance between the crest of a high spot on one roller and the bottom of a depression in a mating roller exceeds the thickness of the paper, such that the paper is not compressed in these areas.

Furthermore, JP-A-59 091 016 discloses the use of rollers to produce a corrugated web. The rollers have mating concave and convex portions in which the clearance between the top and bottom of the respective mating concave and convex roller portions is greater than the thickness of the web material, while the clearance between the sides of the rollers is less than the thickness of the web materials.

The invention provides an improved embossed web according to independent claim 1, a method for embossing webs according to independent claim 6 and an apparatus suitable for producing such webs, i.e. a specific pair of embossing rolls according to independent claim 17. Further advantageous features, aspects and details of the invention are evident from the dependent claims, the description and the drawings. The claims are intended to be understood as a first non-limiting approach of defining the invention in general terms.

The invention is based on the finding that male and female embossing elements having different geometries (unmatched embossing elements) and/or which are made of a relatively deformable material such as rubber or plastic can produce a visually more distinct embossing pattern which imparts greater bulk to the embossed sheet with substantially less loss of firmness compared to conventional embossing techniques. For purposes herein, embossing elements are protrusions (male embossing elements) or depressions (female embossing elements) formed on the surface of a roll or plate used for creating corresponding deflections (embossments) in a web. Common methods of forming embossing elements include engraving or etching.

Hence in one aspect, the invention provides a method for embossing a web comprising deflecting the web between unmatched male and female embossing elements, wherein the sidewall slope of the female embossing elements is greater than the corresponding sidewall slope of the male embossing elements. When the male and female embossing elements engage, the differing sidewall slopes cause the web to be compressed or sheared at the top edge of the female embossing elements as the top edge of the female embossing elements approach being contacted by the corresponding sidewall of the male embossing elements. This results in embossments which are more distinct than conventional embossments and provides greater bulk at a given level of firmness.

In another aspect, the invention provides a method of embossing a tissue web between a pair of embossing rolls, wherein one of the embossing rolls comprises rubber male embossing elements and the other embossing roll comprises rubber female embossing elements. In this aspect, it is preferred that both embossing rolls are rubber covered rolls in which the male and female embossing elements have been laser engraved. It is also preferred that the male and female embossing elements be unmatched.

In another aspect, the invention provides a pair of embossing rolls having unmatched male and female embossing elements, wherein the sidewall slope of the female embossing elements is greater than the corresponding sidewall slope of the male elements. The pair of embossing rolls can include one roll having all male elements and the second roll having all female elements, or each roll can have both male and female elements. Preferably at least one of the embossing rolls, and most preferably both of the embossing rolls, is covered with a rubber surface. Rubber embossing elements yield slightly to the web and are believed to be less likely to damage the strength of the web during embossing.

In a further aspect, the invention provides an embossed web having a plurality of embossments wherein the average thickness of the web is the least at the edge of the embossments. For purposes herein, the edge of the embossments is the area of the embossments adjacent the undeflected plane of the web. This area corresponds generally to the portion of the web which contacts the top edge or corner of the female embossing elements during embossing and will be described further in connection with the drawing. It must be noted that not all webs embossed in accordance with the method of this invention will exhibit reduced thickness at the edge of the embossments in their final product form due to the ability of some webs to recover their shape and thickness after embossing. In this regard, throughdried webs have a greater ability to recover than do wet pressed webs.

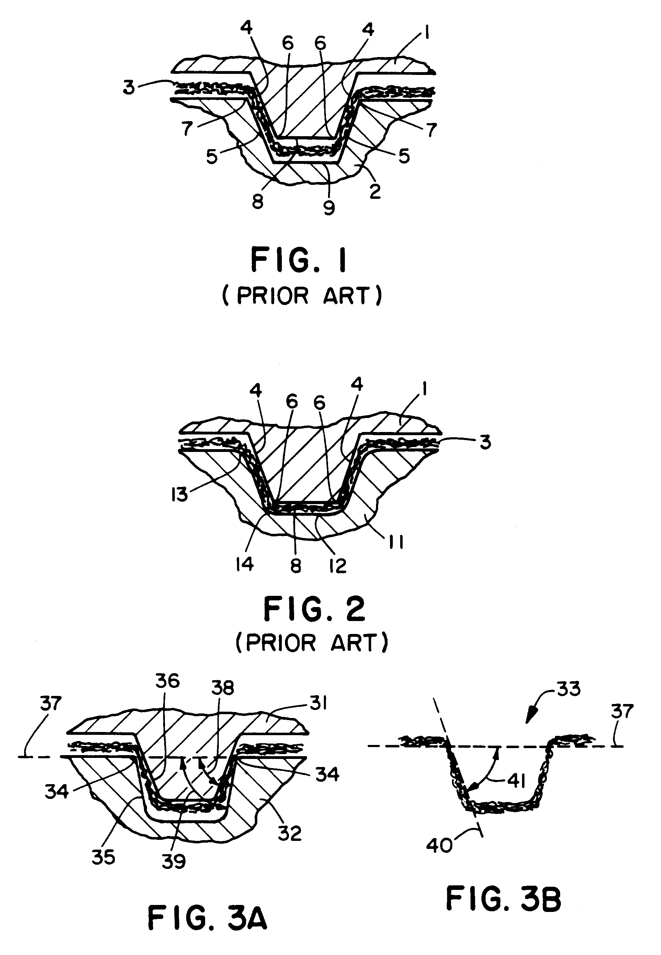

In yet a further aspect the invention provides an embossed web having a plurality of embossments, wherein the average maximum angle of deformation of the embossments is about 45° or greater, preferably about 50° or greater, and most preferably about 55° or greater. The maximum angle of deformation is the maximum angle of the embossment relative to the undeflected plane of the unembossed web. The theoretical maximum angle of deflection is 90°. It is believed that a high maximum angle of deflection is a major factor in the exceptional bulk/firmness relationship which the products of this invention exhibit. This concept will be described in greater detail with respect to Figure 3B.

As indicated above, a feature of this invention pertains to the preference that the male and female embossing elements are "unmatched". As used herein, this term is intended to mean that the male and female embossing elements are not identical in shape, but are positioned relative to each other in registry such that they engage. This is meant to distinguish from conventional "matched" steel embossing elements in which the male elements are engraved first and the female elements are subsequently made from the male elements, or vice versa, so that both elements are virtually inverse or reciprocal images of each other within the practicalities of manufacturing tolerances. This is not the case with the preferred embossing elements of this invention, wherein although the male and female elements fit together or engage reasonably well, the sidewall slopes of the male and female embossing elements differ sufficiently to prevent complete engagement and thereby provide differential compression and/or shear on certain portions of the web when the embossing elements are engaged.

For unmatched embossing elements, it is preferable that the slope of the male element sidewall be from about 5° to about 85°, and more preferably from about 50° to about 70° or greater. It is believed that steeper sidewall angles create a more permanent kink in the web in the vicinity where the embossment meets the undeflected area of the web. Because the depth of the resulting embossment is determined by the extent to which the male embossing element penetrates the female embossing element, the difference in sidewall slopes should not be so great that the penetration or degree of engagement of the male embossing element is insufficient to achieve the desired bulk. Although the degree of difference between the sidewall slope of the male and female elements can vary, it is preferred that the sidewall slope of the male element be at least about 2° less than the corresponding slope of the female element, and more preferably from about 5° to about 10° less than the corresponding slope of the female element. For purposes herein, the sidewall slope is measured relative to the plane of the undeflected web during embossing, with the maximum slope being perpendicular (90°) to the undeflected web. This is described in greater detail below with regard to Figure 3.

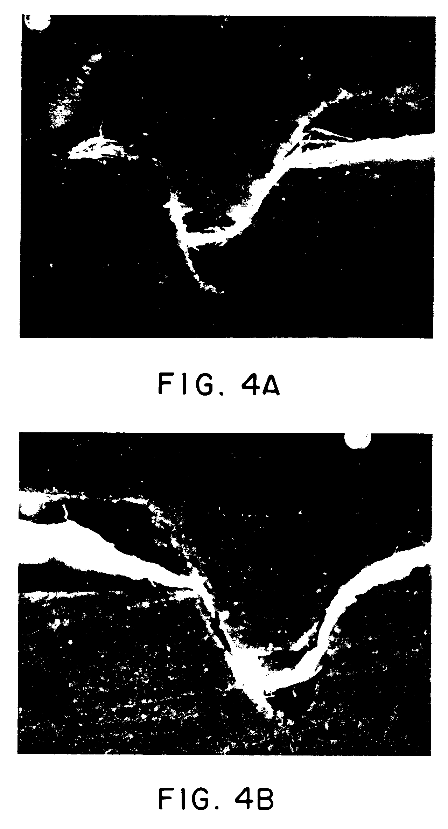

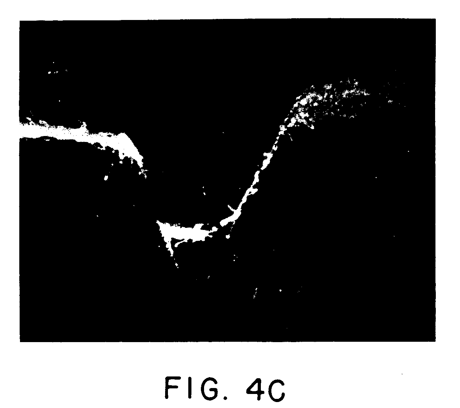

It must be kept in mind that the practicalities of commercial engraving limit the ability to make embossing rolls in which the male and female embossing elements contact each other in the same manner everywhere on the embossing roll or plate. When viewing embossing element engagement in two dimensions, some embossing elements will contact each other on both sides as hereinafter illustrated in Figure 3. In other instances, some embossing elements will contact each other only on one side. In still other areas, the embossing elements may not touch at all. However, overall there are a sufficient and substantial number of contacts to impart exceptional bulk and firmness to the web and integrity to the embossments. The varying degrees of engagement exhibited by the male and female embossing elements in accordance with this invention will be further illustrated and discussed in connection with Figure 4.

When manufacturing embossing rolls for use in connection with this invention, laser engraving is a preferred method of manufacture because of its precision and lower cost. Laser engraved rubber sleeves for embossing rolls and laser engraved plates are commercially available from Midwest Rubber Plate Co., Menasha, Wisconsin. The material of the male and female embossing elements is preferably a deformable material such as rubber, plastic or the like. Such materials are not only more forgiving with respect to degrading the strength of the web during embossing, but they also are suitable for laser engraving. As used herein, "rubber" is meant to include any relatively deformable material having a Shore A hardness of about 100 or less and preferably about 90 or less. Other suitable deformable materials include nylon, polyesters, polyurethane, polytetrafluoroethylene (Teflon), poly(vinylidene fluoride co hexafluoropropylene) (Viton), and the like.

Although deformable embossing element materials are preferred, it is also within the scope of certain aspects of this invention for the embossing elements to be steel or combinations of steel and rubber or other deformable materials. For example, the male elements can be steel and the female elements can be a deformable material, such as rubber, or vice versa. It will be appreciated that many different suitable combinations of materials are possible and within the scope of this invention. The steel rolls of this invention can also be used for bonding nonwovens by heating the rolls to provide a unique bonding pattern.

The web to be embossed in accordance with this invention can be any web suitable for embossing, including paper, tissue, nonwovens, films, laminates, combinations thereof and the like. The webs can be preheated or premoistened. In the case of tissue webs, which for purposes herein means webs intended for use as facial tissue, bath tissue, table napkins and paper towels, the web can be layered or nonlayered, creped or uncreped, wet pressed or throughdried, single-ply or two-ply or multiple ply, and can comprise natural and/or synthetic fibers. Creped tissue webs are preferred, which have a finished dry basis weight of from about 24.45 to 195.6 kg/m$^2$ (about 5 to about 40 pounds per 2880 square feet) per ply and a geometric mean tensile strength of from about 2286 to about 91440 grams per centimeter (about 300 to about 12,000 grams per 3 inches) of width.

For purposes herein, Bulk is the volume of a product (in cubic centimeters) divided by the weight of the product (in grams). For roll products such as bath tissue and paper towels which have a hollow core, the product volume "V" is calculated as follows:

$$V = \pi (R_1{}^2 - R_2{}^2)W$$

wherein

$R_1$ = radius of the roll;
$R_2$ = outside radius of the hollow core; and
W = width of the roll.

For a rectangular stack of product such as facial tissue, the product volume is simply the length x height x width of the stack.

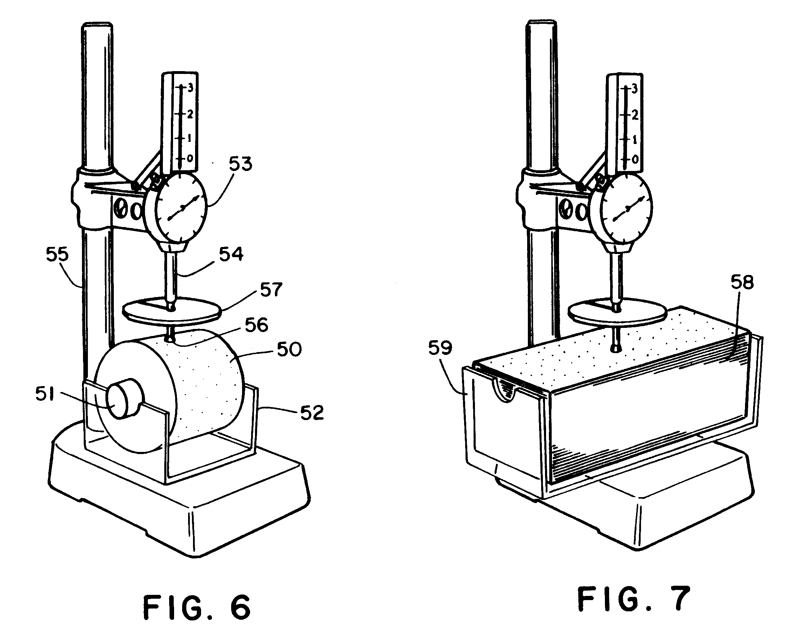

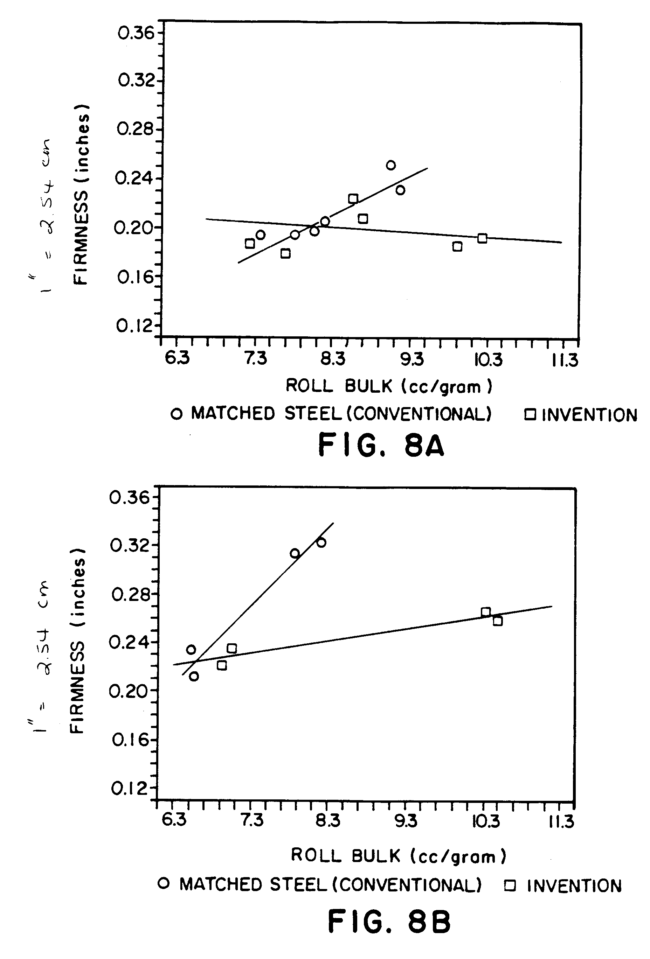

The Firmness Index is a measure of the distance, expressed in centimeters (inches), that a weighted probe deflects the surface of a roll or stack of product. A greater deflection distance and hence a greater Firmness Index reflects lesser product firmness. The method and apparatus for measuring the Firmness Index will be described hereinafter in connection with the detailed description of Figures 7 and 8.

Figure 1 is a schematic illustration of prior art embossing using matched steel embossing elements.

Figure 2 is a schematic illustration of prior art embossing using steel embossing elements against a rubber roll.

Figures 3A and 3B are schematic illustrations of an embossing method in accordance with this invention and the resulting embossed product.

Figures 4A, 4B, and 4C are cross-sectional photographs of a tissue web being embossed between male and female embossing elements in accordance with this invention, illustrating the varying degrees of contact between the male and female elements which can occur within the same embossing pattern.

Figures 5A and 5B are cross-sectional photographs comparing a conventional embossed tissue web and a web in accordance with this invention, respectively.

Figure 6 is an illustration of the apparatus used to measure the Firmness Index for roll products such as bath tissue and paper towels.

Figure 7 is an illustration of essentially the same apparatus illustrated in Figure 6, but modified slightly to measure the Firmness Index of stacked products such as facial tissue and table napkins.

Figures 8A and 8B are plots of roll Bulk vs. roll Firmness Index for embossed bath tissue of this invention compared to the same tissues embossed using matched steel rolls.

Figure 9 represents the particular embossing pattern referred to in the description of the previous Figures.

Referring to the drawing, the invention will be described in greater detail.

Figure 1 illustrates an embossing process of the prior art in which a tissue web is embossed between matched steel embossing elements. Shown is a male steel embossing element 1 and a matching female steel embossing element 2 in an engaged position. The web 3 being embossed is deflected between the embossing elements as shown. The amount of web compression is greatest between the male embossing element sidewalls 4 and the female embossing element sidewalls 5.

Because the sidewall slope of the matched steel male and female embossing elements is identical, the extent of web compression is substantially uniform at all points between the bottom edge 6 of the male element and the top edge 7 of the female element. Under typical embossing conditions, the web is relatively uncompressed in the region between the bottom 8 of the male element and the bottom 9 of the female element.

Figure 2 illustrates another embossing process of the prior art wherein a tissue web 3 is embossed between a male steel embossing element 1 and a rubber embossing surface 11 which partially conforms to the steel male element when engaged as shown. In this type of embossing process, the greatest degree of web compression occurs between the bottom 8 of the steel element and the corresponding surface 12 of the rubber. In the area of the male element sidewalls 4, there is a gradual increase in web compression from a point 13 on the undeflected surface of the rubber to a point 14 near the bottom edge 6 of the male element.

Figure 3A illustrates a preferred embossing method of this invention in which a tissue web 3 is deflected between a male embossing element 31 and an unmatched female embossing element 32. Figure 3B illustrates the resulting embossment 33 of the embossed product. Unlike the prior art embossing methods illustrated in Figures 1 and 2, the greatest degree of web compression and/or shear in the method of this invention occurs in the circled areas 34 located in the vicinity of the top edge of the female element. This occurs because the sidewall slope of the female element sidewall 35 is greater than the sidewall slope of the male element sidewall 36. As previously described, the sidewall slopes are measured relative to the plane of the undeflected web as indicated by dashed line 37. The sidewall slope of the male embossing element as shown in Figure 3A is indicated by the angle represented by the double arrow 38. The sidewall slope of the female embossing element is the angle represented by the double arrow 39. It is preferred that the male and female embossing elements are both made of rubber or other resilient material to provide an embossing surface which is more forgiving than steel and is less likely to cut the web at the point of near contact between the male and female embossing elements.

Figure 3B also illustrates the maximum angle of deformation exhibited by the embossment 33. Shown is the undeflected plane of the web 37 and a line 40 which is aligned parallel with the centerline of the embossment sidewall to give the greatest value for the angle represented by the double arrow 41. Determination of the maximum angle of deformation can readily be done with the naked eye by using a representative cross-sectional photograph of the product.

However, the maximum angle of deflection can also be calculated using image analysis procedures. One method to accomplish this is to input the cross-sectional photograph into an image analyzer with the general center of the cross-section running in the horizontal, or x, direction. The computer image is corrected for shading differences and then is discriminated to create a binary image in which the tissue component is detected. Next, the binary image is manually edited to remove any gross smudges appearing in areas outside of the tissue component. A series of dilations is performed to fill in void areas of the tissue image. Then, an opposite set of erosions is performed to maintain the tissue sheet's contour. Finally, the resulting tissue image is skeletonized to a line of pixels. Precautions must be taken to preserve line endpoints during skeletonization. The image that results from this step is equivalent to a line drawn through the center of the tissue cross-section. Once the skeletonized image is obtained, it may be broken up into line segments. Typically, 32 line segments are created across the y-direction of the image. The angle of the individual line segments is easily measured as the inverse tangent of the ratio of Feret Y over Feret X. The maximum angle of the line segments is a measure of the steepness of the embossment sidewall.

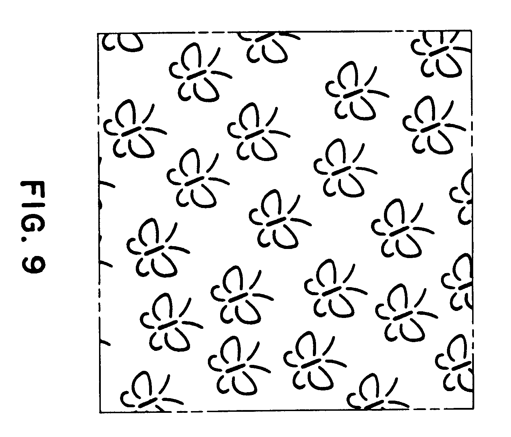

Figures 4A, 4B and 4C are cross-sectional photographs (magnified 25X) of a creped tissue web being embossed between unmatched, laser engraved, rubber embossing elements in accordance with this invention. The embossing pattern was that as illustrated in Figure 9. The cross-sections are taken at different positions of an embossing element to illustrate the manner in which the web is acted upon by the embossing elements in general and the variation in the manner in which the embossing elements engage. The particular embossing elements illustrated were from rubber sleeves adapted to be used on embossing rolls. The dimensions of the male embossing elements, in centimeters (inches), were .15 cm (0.0601 in) in depth, .215 cm (.085 in) wide at the top, and .0635 cm ( 0.025 in) wide at the bottom. The sidewall slope of the male embossing elements was about 60°. The dimensions of the female elements, also in inches, were .127 cm (0.050 in) in depth, .139 cm (0.055 in) wide at the top, and about .038 cm (0.015 in) wide at the bottom, which was rounded. The sidewall slope of the female embossing elements was about 65°. As the photographs clearly show, the effect of using unmatched embossing elements has a variable effect on the web on a micro scale. However, a common effect is the compression or shear of the web at some point along the sidewall of the male embossing element where it approaches contact with the sidewall of the female embossing element. The differing sidewall slopes of the two elements prevent complete engagement and cause the web to be pinched off at some point while being virtually uncompressed at bottom of the male element. For this to occur consistently it is preferred that the maximum width of the male embossing element be at least as great as the maximum width of the female embossing element. Although not shown in these photographs, the relative positions of any given male and female embossing elements also change with time as the embossing elements of the two rolls or sleeves rotate in and out of engagement. Hence, in Figure 4A for example, there apparently was initial contact between the male and female embossing elements on the left side prior to the photograph being taken (note the compression of the web at the top of the female embossing element left sidewall). Hence, when interpreting these photographs, one must not only take into account the imperfections of the laser engraving process, but also the dynamics (rolling action) of the embossing process.

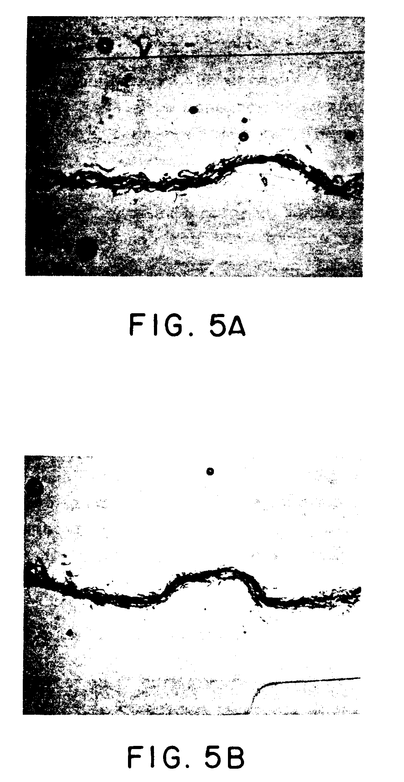

Figures 5A and 5B are cross-sectional photographs (magnified 20X) of a conventional embossed product and a product in accordance with this invention, respectively, using the embossing pattern illustrated in Figure 9. Shown in Figure 5A is a conventional single ply embossed creped tissue which has been embossed between a steel roll and a rubber roll. Figure 5B shows a like web embossed using laser engraved rubber rolls having unmatched male and female embossing elements in accordance with this invention. Note the greater distinctness of the embossment in Figure 5B due to the more sharply angled sidewalls. Also note the relative thickness of the two webs in the area of the edge of the embossments. In the web of Figure 5B, this area is significantly thinner than the other areas along the sidewalls of the embossment. In contrast, the corresponding areas of the conventional embossment of Figure 5A are no thinner than other areas along the embossment sidewalls. This occurs throughout the entire web. It is theorized that applying maximum compression and/or shear only at the edge of the embossment realigns or deforms the fibers in this area in such a manner to give the resulting embossment greater structural integrity. In effect a more permanent crease or bend is imparted to the web at the edge of the embossment which results in greater embossment distinctness and a greater firmness retention for the embossed web.

Figure 6 illustrates the apparatus used to measure the Firmness Index for roll products. The apparatus is available from B.C. Ames Company, Waltham, Massachusetts 02154 and is known as an Ames #16 Bench Comparator. Shown is a roll product 50 being measured which is supported on a core shaft 51, which in turn is supported by a suitable stand 52. A dial indicator 53 (#3223 long range indicator having a 7.6 cm (3 inch) range) mounted on the comparator stand displays the distance of travel of the probe rod 54 and displays readings from 0-100 in increments of 0.001. The length of the post 55 of the comparator stand is 38.1 cm (15 inches) long. The tip of the probe rod is fitted with a contact point 56 having a .515 cm (13/64 inch) diameter and a .43 cm (11/64 inch) radius of curvature (Model P-100 Ames contact point). The probe rod is adapted to be loaded with slotted weight discs 57 to vary the load and resulting travel of the probe rod in deflecting the surface of the product. The total downward force exerted by the probe rod when unloaded is between 70-80 grams.

When carrying out the Firmness Index procedure, the roll product is placed on the core shaft and holder so the indicator probe is approximately centered on the roll, end to end, and hits the apex of the roll curvature. The indicator probe is gently lowered onto the surface crown of the roll and the height of the dial indicator is adjusted until the reading on the dial indicator falls between 3.8 cm (1.5 inches) and 4.8 cm (1.9 inches). The product roll is then rotated to a different center position and the dial indicator reading is recorded after the probe rod has been in contact with the product roll for 15 seconds. This is the first reading. Then, with one hand, the indicator probe is firmly grasped with the thumb and forefinger above the indicator dial using the dial housing for support. With the other hand, a 1 pound weight disc is placed on the indicator probe. The indicator probe is then gently lowered onto the sample roll surface. After the indicator probe has been in contact with the product roll for 15 seconds, the dial indicator reading is taken. This is the second reading. Subtract the second reading from the first reading. The difference is a measure of the roll firmness. Repeat the foregoing procedure at two random positions around the circumference of the product roll, each position being a fixed distance from each end of the roll. For bath tissue, the second and third readings should be 2.5 cm (one inch) from the roll edges. For paper towels, the second and third readings should be 5 cm (two inches) from the roll edges. The average of the three readings is the Firmness Index for the product.

Figure 7 illustrates the apparatus used to measure the Firmness Index for products comprising a stack of webs 58, which is very similar to the apparatus described in regard to Figure 6. The only difference from the apparatus illustrated in Figure 6 is the elimination of the core shaft for supporting the product sample to be measured. Instead, for products comprising a stack of webs, the product stack is simply placed on a suitable tray support 59 or other suitable means which supports the corners of the stack and rests on base of the test apparatus. In most instances the probe rod will be long enough to reach the stack. If not, the product stack can be further supported by any suitable means which raises the stack sufficiently to be measured.

The procedure for determining the Firmness Index for a stack of webs is the same as that described above with regard to roll products, except that the three measurements to be averaged are taken at different locations. The first measurement is taken at the center of the product, as viewed from above. The second and third measurements are taken at diametrically opposite corners of the product, 2.5 cm (one inch) from both edges which form the chosen corner. As before, the average of the three measurements is the Firmness Index for the product stack.

The relationship of roll Firmness Index and roll Bulk for two different embossed webs is illustrated in Figures 8A and 8B, in each case graphically illustrating a significant advantage of this invention compared to conventional embossing. The plot of Figure 8A represents a comparison of a throughdried tissue web which was embossed by matched steel embossing rolls on the one hand and, on the other hand, by laser engraved rubber embossing rolls in accordance with this invention. The embossing pattern for both pairs of embossing rolls was the butterfly pattern illustrated in Figure 9. The basesheet was a one ply, throughdried web having a basis weight of 33 grams per square meter and a geometric mean tensile strength of about 1300 grams per 7.6 cm (3 inches) of width. The furnish was a homogeneous mixture of 60 weight percent softwood bleached kraft and 40 weight percent hardwood bleached kraft papermaking fibers. The matched steel embossing roll elements appeared in cross-section as illustrated in Figure 1 and had a depth of .106 cm (0.042 inch), a base width of .170 cm (0.067 inch), and an apex width of .063 cm (0.025 inch). The laser engraved embossing roll elements appeared in cross-section as illustrated in Figure 4 and had dimensions set forth in the description of Figure 4. In each case, a 40.6 cm (16 inch) wide roll of the basesheet material was unwound, embossed at three different embossing levels, perforated every 11.43 cm (4 1/2 inches) to define individual sheets, and wound onto a 4.12 cm (1 5/8 inch) diameter core to form a log having a 300 sheet count. The log was then cut with a band saw into three product rolls of single-ply bath tissue. For the matched steel embossing, the three different levels of embossing were obtained by three different levels of engagement of the embossing elements: .038 cm (0.015 inch), .05 cm (0.020 inch), and .05 cm (0.024 inch) off bottom. For the laser engraved embossing, the three different levels of embossing were obtained by placing shims between the embossing roll bearing housings and increasing the spacing by .012 cm (0.005 inch) at a time. The initial setting was subjectively determined to give relatively heavy embossing. After the basesheet was embossed at all three conditions, the procedure was replicated to obtain two data points for each condition to improve the confidence level of the data. The results are plotted in Figure 8A.

Figure 8B is a similar plot, but for a different basesheet. The basesheet used for this plot was a wet pressed, two-ply tissue web having a combined basis weight of 31 grams per square meter and a geometric mean tensile strength of about 1100 grams. The furnish was a homogeneous blend of hardwood, softwood, bagasse and secondary papermaking fibers. The procedure and the embossing rolls were otherwise as described above with respect to Figure 8A, except that only two levels of embossing element engagement were tested for the matched steel embossing: .04 cm (0.016 inch) and .03 cm (0.013 inch). The laser engraved embossing rolls were adjusted once by .012 cm (0.005 inch) using shims as described above.

As is illustrated by both plots, as the Bulk is increased the Firmness Index increases for the matched steel product. (An increasing Firmness Index means decreasing firmness.) For the products of this invention, however, the Firmness Index remained relatively constant as the Bulk was increased. Hence by utilizing the method of this invention, one can increase roll Bulk while maintaining a desireable level of roll firmness. At the same time, a more distinct, well-defined embossing pattern is obtained.

**Claims**

1. An embossed web (3) having a plurality of embossments (33) protruding from an undeflected plane surface of the web, said embossments (33) having sloping walls and being substantially deeper than the thickness of the web, wherein the average maximum angle of deformation of the embossments (33) is about 45° or greater, characterized in that the average thickness of the web is the least in the area of the embossments adjacent the undeflected surface of the web (3).

2. The embossed web of Claim 1, wherein the average maximum angle of deformation is about 50° or greater.

3. The embossed web of Claim 1, wherein the average maximum angle of deformation is about 55° or greater.

4. The embossed web of any of Claims 1 to 3, wherein the web is a tissue web.

5. The embossed web of Claim 4, wherein the tissue web is creped.

6. A method for embossing a web, especially for making a web according to one of the preceding claims, comprising deflecting the web between unmatched male and female embossing elements, wherein the sidewall slope of the female embossing elements is greater than the corresponding sidewall slope of the male embossing elements, whereby each sidewall slope is measured relative to the plane of the undeflected web, and wherein the sidewall slope of the male embossing elements is at least about 2° less than the sidewall slope of the female embossing elements.

7. The method of Claim 6, wherein the sidewall slope of the male embossing elements is from 5° to 10° less than the sidewall slope of the female embossing elements.

8. The method of one of Claims 6 or 7, wherein the sidewall slope of the male embossing elements is from 50° to 70°.

9. The method of one of Claims 6 to 8, wherein the embossing elements are a deformable material.

10. The method of one of Claims 6 to 9, wherein the embossing elements are rubber or plastic.

11. The method of one of Claims 6 to 10, wherein the embossing elements are laser engraved.

12. The method of one of Claims 6 to 11, wherein the web is a tissue web.

13. A method for embossing a tissue web according to one of Claims 4 and 5 between a pair of embossing rolls, wherein at least one of the embossing rolls comprises rubber or plastic male embossing elements and the other roll comprises rubber or plastic female embossing elements.

14. The method of Claim 13, wherein both embossing rolls comprise rubber or plastic embossing elements.

15. The method of Claim 13, wherein the embossing elements of both rolls are rubber.

16. The method of one of Claims 14 or 15, wherein the embossing elements of both rolls are laser engraved.

17. A pair of embossing rolls especially for making an embossed web according to one of Claims 1 to 5 or for being used in a process according to one of Claims 6 to 10, the pair of embossing rolls having unmatched male (31) and female (32) embossing elements, wherein the sidewall slope of the female embossing elements (32) is greater than the corresponding sidewall slope of the male embossing elements (31), whereby each sidewall slope is measured relative to the plane of the undeflected web, and wherein the sidewall slope of the male embossing elements is at least about 2° less than the sidewall slope of the female embossing elements.

18. The embossing roll pair of Claim 17, wherein the sidewall slope (38) of the male embossing elements is from about 5° to about 10° less than the sidewall slope (39) of the female embossing elements.

19. The embossing roll pair of one of Claims 17 or 18, wherein the sidewall slope (38) of the male embossing elements is from 50° to 70°.

20. The embossing roll pair of one of Claims 17 to 19, wherein the embossing rolls are plastic or rubber.

21. The embossing roll pair of one of Claims 17 to 20, wherein the embossing rolls are laser engraved.

**Patentansprüche**

1. Geprägte Bahn (3) mit einer Mehrzahl an Prägungen (33), die von einer nicht gebogenen ebenen Oberfläche der Bahn vorstehen, wobei die Prägungen (33) Neigungswände aufweisen und im wesentlichen tiefer als die Dicke der Bahn sind, wobei der durchschnittliche maximale Deformationswinkel der Prägungen (33) etwa 45° oder mehr beträgt, dadurch gekennzeichnet, daß die durchschnittliche Dicke der Bahn im Bereich der Prägungen benachbart zur nicht gebogenen Oberfläche der Bahn (3) am geringsten ist.

2. Geprägte Bahn gemäß Anspruch 1, bei der der durchschnittliche maximale Deformationswinkel etwa 50° oder mehr beträgt.

3. Geprägte Bahn gemäß Anspruch 1, bei der der durchschnittliche maximale Deformationswinkel etwa 55° oder mehr beträgt.

4. Geprägte Bahn gemäß einem der Ansprüche 1 bis 3, bei der die Bahn eine Tissuebahn ist.

5. Geprägte Bahn gemäß Anspruch 4, bei der die Tissuebahn gekreppt ist.

6. Verfahren zur Prägung einer Bahn, insbesondere zur Herstellung einer Bahn gemäß einem der vorhergehenden Ansprüche, das das Biegen der Bahn zwischen nicht passenden männlichen und weiblichen Prägeelementen umfaßt, wobei die Seitenwandneigung der weiblichen Prägeelemente größer ist als die entsprechende Seitenwandneigung der männlichen Prägeelemente, wobei jede Seitenwandneigung relativ zur Ebene der nicht gebogenen Bahn gemessen wird, und wobei die Seitenwandneigung der männlichen Prägeelemente mindestens etwa 2° geringer als die Seitenwandneigung der weiblichen Prägeelemente ist.

7. Verfahren gemäß Anspruch 6, bei dem die Seitenwandneigung der männlichen Prägeelemente 5° bis 10° geringer als die Seitenwandneigung der weiblichen Prägeelemente ist.

8. Verfahren gemäß einem der Ansprüche 6 oder 7, bei dem die Seitenwandneigung der männlichen Prägeelemente 50° bis 70° beträgt.

9. Verfahren gemäß einem der Ansprüche 6 bis 8, bei dem es sich bei den Prägeelementen um ein deformierbares Material handelt.

10. Verfahren gemäß einem der Ansprüche 6 bis 9, bei dem es sich bei den Prägeelementen um Gummi oder Kunststoff handelt.

11. Verfahren gemäß einem der Ansprüche 6 bis 10, bei dem die Prägeelemente lasergraviert sind.

12. Verfahren gemäß einem der Ansprüche 6 bis 11, bei dem die Bahn eine Tissuebahn ist.

13. Verfahren zur Prägung einer Tissuebahn gemäß einem der Ansprüche 4 oder 5 zwischen einem Paar Prägewalzen, wobei mindestens eine der Prägewalzen männliche Gummi- oder Kunststoffprägeelemente und die andere Walze weibliche Gummi- oder Kunststoffprägeelemente aufweist.

14. Verfahren gemäß Anspruch 13, bei dem beide Prägewalzen Gummi- oder Kunststoffprägeelemente aufweisen.

15. Verfahren gemäß Anspruch 13, bei dem es sich bei den Prägeelementen beider Walzen um Gummi handelt.

16. Verfahren gemäß einem der Ansprüche 14 oder 15, bei dem die Prägeelemente beider Walzen lasergraviert sind.

17. Prägewalzenpaar, insbesondere zur Herstellung einer geprägten Bahn gemäß einem der Ansprüche 1 bis 5 oder zur Verwendung in einem Verfahren gemäß einem der Ansprüche 6 bis 10, wobei das Prägewalzenpaar nicht passende männliche (31) und weibliche (32) Prägeelemente aufweist, wobei die Seitenwandneigung der weiblichen Prägeelemente (32) größer ist als die entsprechende Seitenwandneigung der männlichen Prägeelemente (31), wobei jede Seitenwandneigung relativ zur Ebene der nicht gebogenen Bahn gemessen wird, und wobei die Seitenwandneigung der männlichen Prägeelemente mindestens etwa 2° geringer als die Seitenwandneigung der weiblichen Prägeelemente ist.

18. Prägewalzenpaar gemäß Anspruch 17, bei dem die Seitenwandneigung (38) der männlichen Prägeelemente etwa 5° bis etwa 10° weniger als die Seitenwandneigung (39) der weiblichen Prägeelemente beträgt.

19. Prägewalzenpaar gemäß einem der Ansprüche 17 oder 18, bei dem die Seitenwandneigung (38) der männlichen Prägeelemente 50° bis 70° beträgt.

20. Prägewalzenpaar gemäß einem der Ansprüche 17 bis 19, bei dem es sich bei den Prägewalzen um Kunststoff oder Gummi handelt.

21. Prägewalzenpaar gemäß einem der Ansprüche 17 bis 20, bei dem die Prägewalzen lasergraviert sind.

## Revendications

1. Nappe gaufrée (3) ayant une pluralité de gaufrages (33) faisant saillie depuis une surface plane non déformée de la nappe, lesdits gaufrages (33) ayant des parois en pente et étant sensiblement plus profonds que l'épaisseur de la nappe, l'angle maximum moyen de déformation des gaufrages (33) étant d'environ 45° ou plus,

   caractérisée en ce que

   l'épaisseur moyenne de la nappe est la plus faible dans la zone des gaufrages adjacente à la surface non déformée de la nappe (3).

2. Nappe gaufrée selon la revendication 1, dans laquelle l'angle maximum moyen de déformation est d'environ 50° ou plus.

3. Nappe gaufrée selon la revendication 1, dans laquelle l'angle maximum moyen de déformation est d'environ 55° ou plus.

4. Nappe gaufrée selon l'une quelconque des revendications 1 à 3, qui est une nappe de papier absorbant mince.

5. Nappe gaufrée selon la revendication 1, dans laquelle la nappe de papier absorbant mince est crêpée.

6. Procédé de gaufrage d'une nappe, en particulier pour fabriquer une nappe selon l'une quelconque des revendications précédentes, qui consiste à déformer la nappe entre des éléments de gaufrage mâles et femelles non appariés, procédé dans lequel la pente de la paroi latérale des éléments de gaufrage femelles est supérieure à la pente de la paroi latérale correspondante des éléments de gaufrage mâles, chaque pente de paroi latérale étant mesurée par rapport au plan de la nappe non déformée, et selon lequel la pente de la paroi latérale des éléments de gaufrage mâles est inférieure d'au moins 2° environ à la pente de la paroi latérale des éléments de gaufrage femelles.

7. Procédé selon la revendication 6, dans lequel la pente de la paroi latérale des éléments de gaufrage mâles est inférieure de 5 à 10° à la pente de la paroi latérale des éléments de gaufrage femelles.

8. Procédé selon l'une des revendications 6 à 7, dans lequel la pente de la paroi latérale des éléments de gaufrage mâles est d'environ 50° à 70°.

9. Procédé selon l'une des revendications 6 à 8, dans lequel les éléments de gaufrage sont en un matériau déformable.

10. Procédé selon l'une des revendications 6 à 9, dans lequel les éléments de gaufrage sont en caoutchouc ou en plastique.

11. Procédé selon l'une des revendications 6 à 10, dans lequel les éléments de gaufrage sont gravés au laser.

12. Procédé selon l'une des revendications 6 à 11, dans lequel la nappe est une nappe de papier absorbant mince.

13. Procédé de gaufrage d'une nappe en papier absorbant mince selon l'une quelconque des revendications 4 à 5 entre une paire de rouleaux de gaufrage, dans lequel l'un au moins des rouleaux de gaufrage comporte des éléments de gaufrage mâles en caoutchouc ou en plastique et l'autre rouleau comprend des éléments de gaufrage femelles en caoutchouc ou en plastique.

14. Procédé selon la revendication 13, dans lequel les deux rouleaux de gaufrage comprennent des éléments de gaufrage en caoutchouc ou en plastique.

15. Procédé selon la revendication 13, dans lequel les éléments de gaufrage des deux rouleaux sont en caoutchouc.

16. Procédé selon la revendication 14 ou 15, dans lequel les éléments de gaufrage des deux rouleaux sont gravés au laser.

17. Paire de rouleaux de gaufrage, en particulier pour la fabrication d'une nappe gaufrée selon l'une quelconque des revendications 1 à 5 ou pour être utilisée dans le procédé selon l'une des revendications 6 à 10, ladite paire de rouleaux de gaufrage ayant des éléments de gaufrage mâles (31) et femelles (32) non appariés, paire dans laquelle la pente de la paroi latérale des éléments de gaufrage femelles (32) est supérieure à la pente de la paroi latérale correspondante des éléments de gaufrage mâles (31), chaque pente de paroi latérale étant mesurée par rapport au plan de la nappe non déformée, et dans laquelle la pente de la paroi latérale des éléments de gaufrage mâles est inférieure d'au moins environ 2° à la pente de la paroi latérale des éléments de gaufrage femelles.

18. Paire de rouleaux de gaufrage selon la revendication 17, dans laquelle la pente de la paroi latérale (38) des éléments de gaufrage mâles est inférieure d'environ 5 à environ 10° à la pente de la paroi latérale (39) des éléments de gaufrage femelles.

19. Paire de rouleaux de gaufrage selon la revendication 17 ou 18, dans laquelle la pente de la paroi latérale (38) des éléments de gaufrage mâles est comprise entre 50° et 70°.

20. Paire de rouleaux de gaufrage selon l'une des revendications 17 à 19, dans laquelle les rouleaux de gaufrage sont en plastique ou en caoutchouc.

21. Paire de rouleaux de gaufrage selon l'une des revendications 17 à 20, dans laquelle les rouleaux de gaufrage sont gravés au laser.

FIG. 1
(PRIOR ART)

FIG. 2
(PRIOR ART)

FIG. 3A

FIG. 3B

FIG. 4A

FIG. 4B

FIG. 4C

FIG. 5A

FIG. 5B

FIG. 7

FIG. 6

**FIG. 8A**

**FIG. 8B**

FIG. 9